(19) Europäisches Patentamt
European Patent Office
Office européen des brevets

(11) Veröffentlichungsnummer: **0 325 131 A2**

(12) **EUROPÄISCHE PATENTANMELDUNG**

(21) Anmeldenummer: **89100252.9**

(51) Int. Cl.⁴: **C07D 239/42 , A23K 1/16**

(22) Anmeldetag: **09.01.89**

(30) Priorität: **20.01.88 DE 3801521**

(43) Veröffentlichungstag der Anmeldung:
**26.07.89 Patentblatt 89/30**

(84) Benannte Vertragsstaaten:
**BE CH DE ES FR GB IT LI NL**

(71) Anmelder: **BAYER AG**

**D-5090 Leverkusen 1 Bayerwerk(DE)**

(72) Erfinder: **Bonse, Gerhard, Dr.**
**Wolfskaul 3**
**D-5000 Köln 80(DE)**
Erfinder: **Hallenbach, Werner, Dr.**
**Kleiststrasse 10**
**D-4018 Langenfeld(DE)**
Erfinder: **Lindel, Hans, Dr.**
**Carl-Duisberg-Strasse 321**
**D-5090 Leverkusen 1(DE)**
Erfinder: **Berschauer, Friedrich Prof. Dr.**
**Claudiusweg 9**
**D-5600 Wuppertal 1(DE)**
Erfinder: **Greife, Heinrich, A. Dr.**
**Salmstrasse 23**
**D-4018 Langenfeld(DE)**
Erfinder: **Klotz, Gernot, Dr.**
**Förster-Sons-Strasse 32**
**D-5653 Leichlingen 1(DE)**

(54) **Pyrimidinderivate als Leistungsförderer.**

(57) Die vorliegende Erfindung betrifft neue Pyrimidinderivate der Formel I

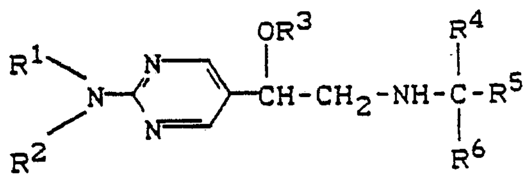

(I)

in welcher
$R^1$ für Wasserstoff, Alkyl, Acyl steht,
$R^2$ für Wasserstoff, Alkyl steht,
$R^1$ und $R^2$ können gemeinsam mit dem Stickstoffatom, an das sie gebunden sind, einen gesättigten oder ungesättigten 5-Ring oder 6-Ring bilden, der noch weitere Heteroatome enthalten kann,
$R^3$ für Wasserstoff, Alkyl, Acyl steht,
$R^4$ für Wasserstoff, Alkyl, Halogenalkyl steht,
$R^5$ für Alkyl, Halogenalkyl steht,
$R^6$ für Alkyl oder Cycloalkyl steht, die gegebenenfalls substituiert sind durch Halogen, Cycloalkyl, Hydroxy, Alkoxy, Halogenalkoxy, Aryl, das seinerseits substituiert sein kann durch Alkyl, Halogen, Hydroxy, Alkoxy, Alkylendioxy, CN oder die Reste $-COR^7$, $-O-Alkylen-COR^7$, Alkylen-$R^8$ oder O-Alkylen-$R^8$,
$R^7$ für Hydroxy, Alkoxy oder $-NR^9R^{10}$ steht,
$R^8$ für Hydroxy, Alkoxy oder $-NR^9R^{10}$ steht,
$R^9$ für Wasserstoff, Alkyl steht,
$R^{10}$ für Wasserstoff, Alkyl steht,
sowie deren tautomere Formen für den Fall, daß mindestens einer der Reste $R^1$ oder $R^2$ für Wasserstoff

EP 0 325 131 A2

steht, und physiologisch verträglichen Salze sowie ihre Herstellung.
Die Verbindungen der Formel I werden als Leistungsförderer für Tiere verwendet.

## Pyrimidinderivate als Leistungsförderer

Die vorliegende Erfindung betrifft neue Pyrimidinderivate, ihre Verwendung als Leistungsförderer für Tiere, sowie Zwischenprodukte und Verfahren zu ihrer Herstellung.

1-Pyridyl-2-aminoethanole und ihre Eignung als Leistungsförderer für Tiere sind bekannt. Ihre Wirkung befriedigt jedoch nicht in jedem Fall (EP-OS 170 538).

Gegenstand der vorliegenden Erfindung sind

1. Pyrimidinderivate der Formel I

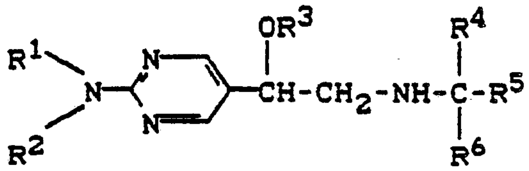

(I)

in welcher
$R^1$ für Wasserstoff, Alkyl, Acyl steht,
$R^2$ für Wasserstoff, Alkyl steht,
$R^1$ und $R^2$ können gemeinsam mit dem Stickstoffatom, an das sie gebunden sind, einen gesättigten oder ungesättigten 5-Ring oder 6-Ring bilden, der noch weitere Heteroatome enthalten kann,
$R^3$ für Wasserstoff, Alkyl, Acyl steht,
$R^4$ für Wasserstoff, Alkyl, Halogenalkyl steht,
$R^5$ für Alkyl, Halogenalkyl steht,
$R^6$ für Alkyl oder Cycloalkyl steht, die gegebenenfalls substituiert sind durch Halogen, Cycloalkyl, Hydroxy, Alkoxy, Halogenalkoxy, Aryl, das seinerseits substituiert sein kann durch Alkyl, Halogen, Hydroxy, Alkoxy, Alkylendioxy, CN oder die Reste $-COR^7$, $-O-Alkylen-COR^7$, Alkylen-$R^8$ oder O-Alkylen-$R^8$,
$R^7$ für Hydroxy, Alkoxy oder $-NR^9R^{10}$ steht,
$R^8$ für Hydroxy, Alkoxy oder $-NR^9R^{10}$ steht,
$R^9$ für Wasserstoff, Alkyl steht,
$R^{10}$ für Wasserstoff, Alkyl steht,
sowie deren tautomere Formen für den Fall, daß mindestens einer der Reste $R^1$ oder $R^2$ für Wasserstoff steht.

2. Verfahren zur Herstellung der neuen Pyrimidinderivate der Formel I

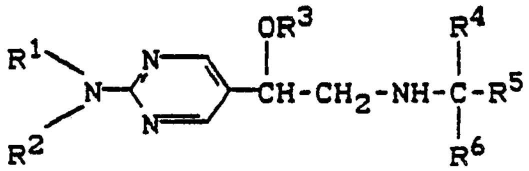

(I)

in welcher
$R^1$ für Wasserstoff, Alkyl, Acyl steht,
$R^2$ für Wasserstoff, Alkyl steht,
$R^1$ und $R^2$ können gemeinsam mit dem Stickstoffatom, an das sie gebunden sind, einen gesättigten oder ungesättigten 5-Ring oder 6-Ring bilden, der noch weitere Heteroatome enthalten kann,
$R^3$ für Wasserstoff, Alkyl, Acyl steht,
$R^4$ für Wasserstoff, Alkyl, Halogenalkyl steht,
$R^5$ für Alkyl, Halogenalkyl steht,
$R^6$ für Alkyl oder Cycloalkyl steht, die gegebenenfalls substituiert sind durch Halogen, Cycloalkyl, Hydroxy, Alkoxy, Halogenalkoxy, Aryl, das seinerseits substituiert sein kann durch Alkyl, Halogen, Hydroxy, Alkoxy, Alkylendioxy, CN oder die Reste $-COR^7$, $-O-Alkylen-COR^7$, Alkylen-$R^8$ oder O-Alkylen-$R^8$,
$R^7$ für Hydroxy, Alkoxy oder $-NR^9R^{10}$ steht,
$R^8$ für Hydroxy, Alkoxy oder $-NR^9R^{10}$ steht,
$R^9$ für Wasserstoff, Alkyl steht,
$R^{10}$ für Wasserstoff, Alkyl steht,

sowie deren Tautomere und physiologisch verträgliche Salze, dadurch gekennzeichnet, daß man

a) Halogenmethylketone der Formel II

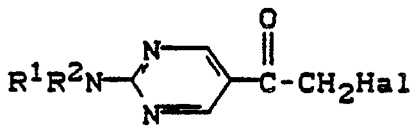

(II)

in welcher
$R^1$ und $R^2$ die oben angegebene Bedeutung haben und
Hal für Halogen steht,
mit Aminen der Formel III

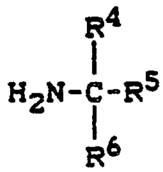

(III)

in welcher
$R^4$ bis $R^6$ die oben angegebene Bedeutung haben,
umsetzt und anschließend die Carbonylgruppe reduziert oder

b) β-Halogenethylverbindungen der Formel IV

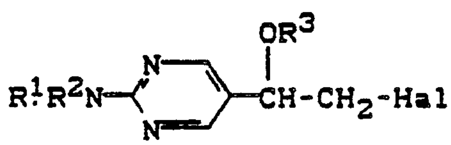

(IV)

in welcher
$R^1$ bis $R^3$ die oben angegebene Bedeutung haben und
Hal für Halogen steht,
mit Aminen der Formel III

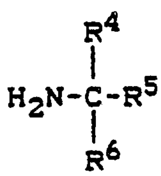

(III)

in welcher
$R^4$ bis $R^6$ die oben angegebene Bedeutung haben,
umsetzt oder

c) für den Fall, daß in Formel I $R^4$ für Wasserstoff steht, indem man Verbindungen der Formel V

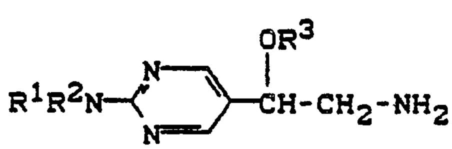

(V)

in welcher
$R^1$ bis $R^3$ die oben angegebene Bedeutung haben
mit Verbindungen der Formel VI

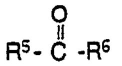 (VI)

in welcher $R^5$ und $R^6$ die oben angegebene Bedeutung haben, unter reduzierenden Bedingungen umsetzt oder

d) Verbindungen der Formel VII

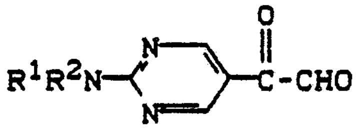

(VII)

in welcher
$R^1$ und $R^2$ die oben angegebene Bedeutung haben,
mit Aminen der Formel III

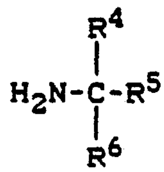

(III)

in welcher
$R^4$ bis $R^6$ die oben angegebene Bedeutung haben,
unter reduzierenden Bedingungen umsetzt.

3. Neue Verbindungen der Formel II

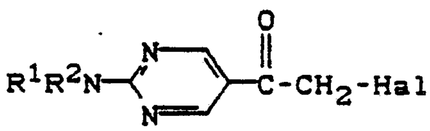

(II)

in welcher
$R^1$ für Wasserstoff, Alkyl, Acyl,
$R^2$ für Wasserstoff, Alkyl,
Hal für Halogen steht,

4. Verfahren zur Herstellung der neuen Verbindungen der Formel II gemäß (3), dadurch gekennzeichnet, daß man Acetylverbindungen der Formel VIII

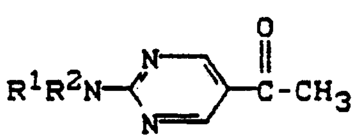

(VIII)

in welcher
$R^1$ für Wasserstoff, Alkyl, Acyl,
$R^2$ für Wasserstoff, Alkyl steht
a) mit elementarem Halogen,
b) mit Kupferhalogeniden der Formel $CuHal_2$ umsetzt.

5. Neue Verbindungen der Formel IV

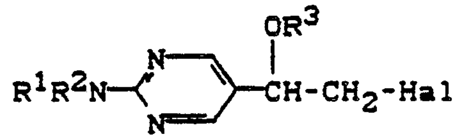

(IV)

in welcher
$R^1$ für Wasserstoff, Alkyl, Acyl steht,
$R^2$ für Wasserstoff, Alkyl steht,
$R^3$ für Wasserstoff, Alkyl, Acyl steht.

6. Verfahren zur Herstellung der neuen Verbindungen der Formel IV gemäß (5), dadurch gekennzeichnet, daß man Verbindungen der Formel II

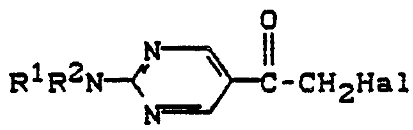

(II)

in welcher
$R^1$, $R^2$ und Hal die bei (5) angegebenen Bedeutungen haben,
reduziert und gegebenenfalls alkyliert oder acyliert.

7. Neue Verbindungen der Formel V

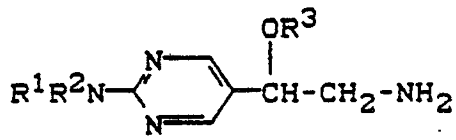

(V)

in welcher
$R^1$ für Wasserstoff, Alkyl, Acyl steht,
$R^2$ für Wasserstoff, Alkyl steht,
$R^3$ für Wasserstoff, Alkyl, Acyl steht.

8. Verfahren zur Herstellung der neuen Verbindungen der Formel V gemäß (7), dadurch gekennzeichnet, daß man

a) Verbindungen der Formel IX

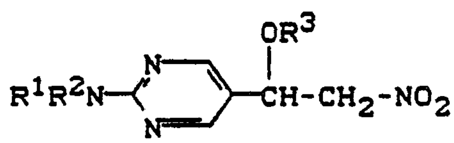

(IX)

in welcher
$R^1$ bis $R^3$ die bei (7) angegebene Bedeutung haben,
katalytisch reduziert,

b) für den Fall, daß $R^3$ für Wasserstoff steht, indem man Verbindungen der Formel X

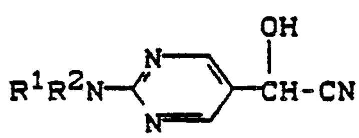

(X)

in welcher
$R^1$ und $R^2$ die bei (7) angegebene Bedeutung haben,
reduziert.

9. Neue Verbindungen der Formel VII

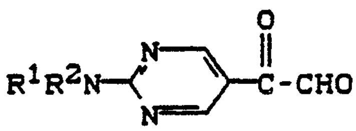

(VII)

in welcher
$R^1$ für Wasserstoff, Alkyl, Acyl steht,
$R^2$ für Wasserstoff, Alkyl steht.

10. Verfahren zur Herstellung der Verbindungen der Formel VII gemäß (9), dadurch gekennzeichnet, daß man Verbindungen der Formel II

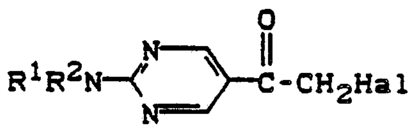

(II)

in welcher
$R^1$ und $R^2$ die bei (9) angegebene Bedeutung haben und
Hal für Halogen steht,
oxidiert.

11. Neue Verbindungen der Formel VIII

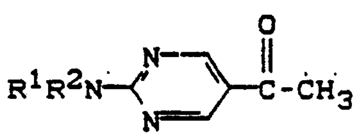

(VIII)

in welcher
$R^1$ für Wasserstoff, Alkyl, Acyl steht,
$R^2$ für Wasserstoff, Alkyl steht.

12. Verfahren zur Herstellung der Verbindungen der Formel VIII gemäß (11), dadurch gekennzeichnet, daß man Verbindungen der Formel XI

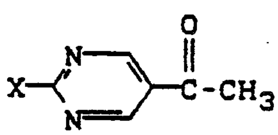

(XI)

in welcher
X für Halogen, $-SO_2$-Alkyl, $-SO_2$-Aryl steht,
mit Aminen der Formel XII
$HNR^1R^2$ (XII)
in welcher
$R^1$ und $R^2$ die bei (11) angegebene Bedeutung haben,
umsetzt.

13. Neue Verbindungen der Formel IX

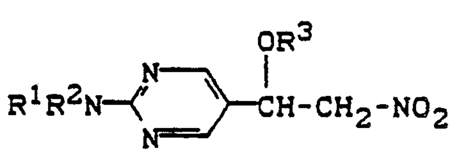

(IX)

in welcher
$R^1$ für Wasserstoff, Alkyl, Acyl steht,
$R^2$ für Wasserstoff, Alkyl steht,
$R^3$ für Wasserstoff, Alkyl, Acyl steht.

14. Verfahren zur Herstellung der Verbindungen der Formel IX gemäß (13), dadurch gekennzeichnet, daß man Verbindungen der Formel XIII

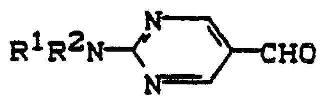

(XIII)

in welcher
$R^1$ und $R^2$ die bei (13) angegebene Bedeutung haben,
mit Nitromethan umsetzt und gegebenenfalls anschließend alkyliert und acyliert.

15. Neue Verbindungen der Formel X

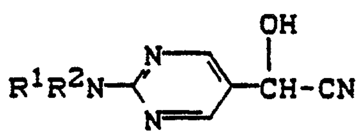

(X)

in welcher
$R^1$ für Wasserstoff, Alkyl, Acyl steht,
$R^2$ für Wasserstoff, Alkyl steht.

16. Verfahren zur Herstellung der Verbindungen der Formel X gemäß (15), dadurch gekennzeichnet, daß man Verbindungen der Formel XIII

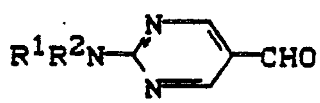

(XIII)

in welcher
$R^1$ und $R^2$ die bei (15) angegebene Bedeutung haben,
mit Blausäure oder deren Salzen umsetzt.

17. Neue Verbindungen der Formel XIII

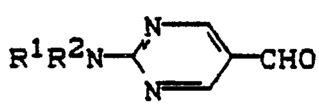

(XIII)

in welcher
$R^1$ für Wasserstoff, Alkyl, Acyl steht,
$R^2$ für Wasserstoff, Alkyl steht.

18. Verfahren zur Herstellung der Verbindungen der Formel XIII gemäß (17), dadurch gekennzeichnet, daß man Verbindungen der Formel XIV

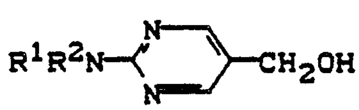

(XIV)

in welcher
$R^1$ und $R^2$ die bei (17) angegebene Bedeutung haben,
oxidiert.

19. Neue Verbindungen der Formel XIV

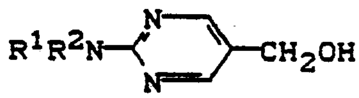

(XIV)

in welcher
$R^1$ für Wasserstoff, Alkyl, Acyl steht,
$R^2$ für Wasserstoff, Alkyl steht.

20. Verfahren zur Herstellung der Verbindungen der Formel XIV gemäß (19), dadurch gekennzeichnet, daß man Verbindungen der Formel XV

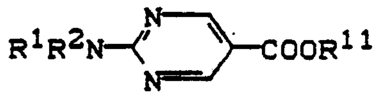

(XV)

in welcher
$R^1$ und $R^2$ die bei (19) angegebene Bedeutung haben, und
$R^{11}$ für H oder Alkyl steht,
reduziert.

Verbindungen der Formel I lassen sich als Leistungsförderer bei Tieren und besonders zur Förderung des Wachstums bei Tieren einsetzen.

Die Verbindungen der Formel I können auch in Form ihrer Racemate sowie als Gemische der zueinander diastereomeren oder enantiomeren Formen vorliegen.

Physiologisch verträgliche Salze der Verbindungen der Formel I können mit folgenden Säuren gebildet werden:

Salzsäure, Schwefelsäure, Phosphorsäure, Perchlorsäure, Brom-, Iodwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäuren, Ameisensäure, Toluolsulfonsäure, Benzolsulfonsäure, Phthalsäure, Naphthalinsulfonsäure, Nikotinsäure, Palmitinsäure, Embonsäure.

Bevorzugt sind Verbindungen der Formel I, in welcher
$R^1$ für Wasserstoff, $C_1$-$C_4$-Alkyl, Acetyl steht,
$R^2$ für Wasserstoff, $C_1$-$C_4$-Alkyl steht,
$R^3$ für Wasserstoff, $C_1$-$C_4$-Alkyl, Acetyl steht,
$R^4$ für Wasserstoff, $C_1$-$C_4$-Alkyl steht,
$R^5$ für $C_1$-$C_4$-Alkyl steht,
$R^6$ für $C_{1-4}$-Alkyl steht, das gegebenenfalls substituiert ist durch Halogen, OH, $C_{1-4}$-Alkoxy, $C_{1-4}$-Halogenalkoxy, Phenyl, gegebenenfalls substituiert durch $C_{1-4}$-Alkyl, $COR^7$, -O-$C_{1-3}$-Alkylen-$COR^7$, $C_{1-3}$-Alkylen-$R^8$, O-$C_{1-3}$-Alkylen-$R^8$,
$R^7$ für OH, $C_{1-4}$-Alkoxy, Amino, Methylamino, Dimethylamino steht
$R^8$ für OH, $C_{1-4}$-Alkoxy, Amino, Methylamino, Dimethylamino steht.

Besonders bevorzugt sind Verbindungen der Formel I, in welcher
$R^1$ für Wasserstoff steht,
$R^2$ für Wasserstoff steht,
$R^3$ für Wasserstoff steht,
$R^4$ für Wasserstoff oder Methyl steht,
$R^5$ für Methyl steht,
$R^6$ für Methyl steht, das gegebenenfalls substituiert ist durch Hydroxy oder Phenyl, das seinerseits substituiert ist durch Hydroxyethyloxy, Methoxyethyloxy, Methoxycarbonylmethyloxy.

Im einzelnen seien neben den Beispielen die folgenden Verbindungen der Formel I genannt:

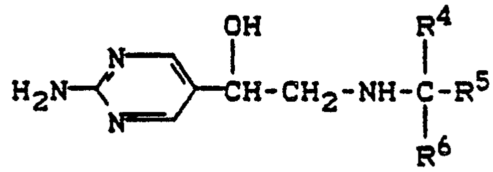

| $R^4$ | $R^5$ | $R^6$ |
|---|---|---|
| H | $CH_3$ | $CH_3$ |
| $CH_3$ | $CH_3$ | $CH_2OH$ |
| H | $CH_3$ | $CH_2C_6H_4OCH_2CH_2OH$(p) |
| H | $CH_3$ | $CH_2C_6H_4OCH_2COOCH_3$(p) |
| H | $CH_3$ | $CH_2C_6H_4OCH_2CH_2N(CH_3)_2$(p) |

Die neuen Verbindungen der Formel I lassen sich nach den unter 2 a - d angegebenen Verfahren herstellen.

Setzt man bei Verfahren 2a) als Verbindung der Formel II 2-Aminopyrimidinyl-5-brommethylketon und als Amin der Formel III Isopropylamin ein, läßt sich Verfahren 2a) durch folgendes Schema wiedergeben:

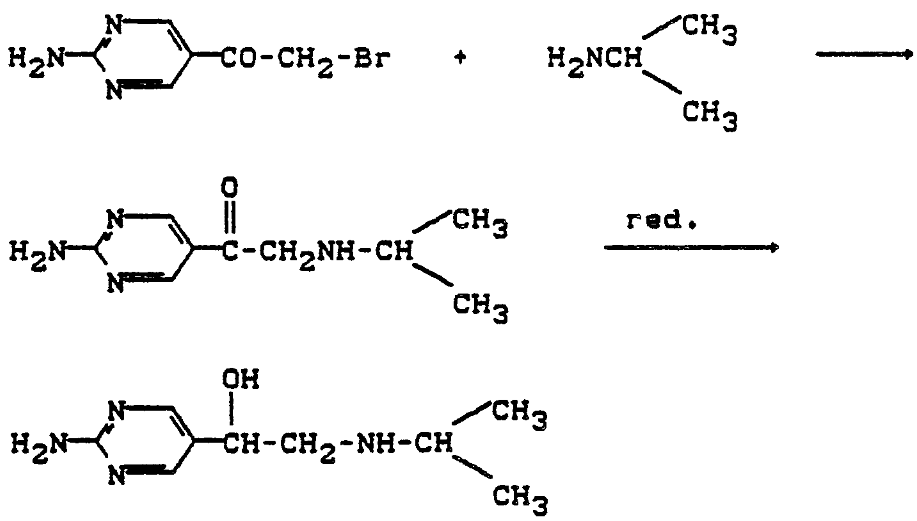


Die Verbindungen der Formel II sind neu. Ihre Herstellung wird weiter unten beschrieben. Die Substituenten $R^1$ und $R^2$ besitzen bevorzugt die weiter oben angegebenen bevorzugten Bedeutungen. Im einzelnen seien folgende Verbindungen der Formel II genannt:

(2-Amino-5-pyrimidinyl)-brommethylketon
(2-Amino-5-pyrimidinyl)-chlormethylketon
(2-Methylamino-5-pyrimidinyl)-brommethylketon
(2-Acetamino-5-pyrimidinyl)-chlormethylketon

Die Amine der Formel III sind bekannt (vergl. z. B. EP-OS 23 385). Die Substituenten $R^4$ bis $R^6$ besitzen bevorzugt die weiter oben bei den Verbindungen der Formel I genannten bevorzugten Bedeutungen. Im einzelnen seien folgende Amine der Formel III genannt:

Isopropylamin,
Isobutylamin,
sec. Butylamin,
tert. Butylamin,
2-Amino-2-methyl-propanol-1,
3-(4-Carbomethoxyphenyl)-2-propylamin,
3-(4-Methoxycarbonylmethoxyphenyl)-2-propylamin,
3-(4-Carboxyphenyl)-2-propylamin,

3-(4-Carboxymethoxyphenyl)-2-propylamin,
3-(4-Hydroxymethylphenyl)-2-propylamin,
3-(4-Dimethylaminomethylphenyl)-2-propylamin,
3-[4-(2-Hydroxyethyloxy)phenyl]-2-propylamin.

Als Reduktionsmittel zur Durchführung des Verfahrens 2a) seien folgende Reduktionsmittel genannt: $H_2$/Katalysator, als Katalysator seien beispielsweise genannt: $PtO_2$, Pd-Aktivkohle; komplexe Metallhydride wie z. B. $LiAlH_4$, $NaBH_4$, $NaBH_3CN$.

Besonders bevorzugt werden folgende Reduktionsmittel eingesetzt: $NaBH_4$ und $NaBH_3CN$

Verfahren 2a) wird durchgeführt, indem man die Verbindungen II und III in einem Verdünnungsmittel in etwa äquimolarem Verhältnis zusammengibt und anschließend reduziert.

Die Reduktion wird bei Temperaturen von -20° C bis +100° C bevorzugt durchgeführt.

Es wird bevorzugt bei Normaldruck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Petrolether, Ligroin, Benzol, Toluol; chlorierte Kohlenwasserstoffe wie Methylenchlorid, Ethylenchlorid, Chloroform; Ether wie Diethylether und Glykoldimethylether; Nitrile wie Acetonitril, Propionitril und Benzonitril; Alkohole wie Methanol, Ethanol, n- und i-Propanol.

Bevorzugt sind Alkohole, wobei ohne Isolierung der Zwischenstufen sofort die Reduktion durchgeführt werden kann.

Setzt man bei Verfahren 2b) als Verbindung der Formel IV 1-(2-Methylamino-5-pyrimidinyl)-2-chlorethanol und als Amin der Formel III 3-(4-Carbomethoxyphenyl)-2-propylamin ein, läßt sich das Verfahren 2b) durch folgendes Schema wiedergeben:

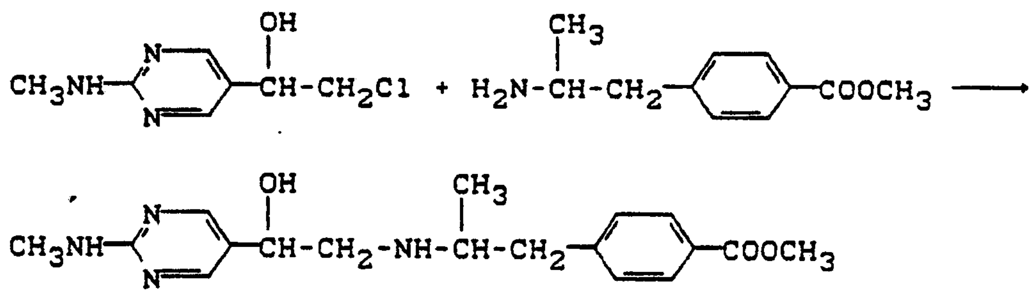

β-Halogenethylverbindungen der Formel IV sind neu. Ihre Herstellung wird weiter unten beschrieben. Die Substituenten $R^1$ bis $R^3$ besitzen bevorzugt die bei den Verbindungen der Formel I angegebenen bevorzugten Bedeutungen. Im einzelnen seien folgende Verbindungen der Formel IV genannt:
1-(2-Amino-5-pyrimidinyl)-2-chlorethanol
1-(2-Amino-5-pyrimidinyl)-2-bromethanol
1-(2-Methylamino-5-pyrimidinyl)-2-chlorethanol
1-(2-Acetamino-5-pyrimidinyl)-2-bromethanol

Verfahren 2b) wird durchgeführt, indem man die β-Halogenethylverbindung der Formel IV mit überschüssigem Amin der Formel III, gegebenenfalls in Gegenwart eines Verdünnungsmittels umsetzt.

Die Reaktion wird bei Temperaturen von +20 bis +150° C durchgeführt.

Es wird bei Normaldruck oder unter erhöhtem Druck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Cyclohexan, Benzol, Toluol, Methylenchlorid, Chloroform, ferner Ether wie Diethylether, Tetrahydrofuran und Dioxan, weiterhin Nitrile wie Acetonitril und Benzonitril, ferner Amide wie Dimethylformamid, ferner Alkohole wie Methanol, Ethanol, n- und i-Propanol.

Bevorzugt eingesetzt werden Alkohole.

Setzt man bei Verfahren 2c) als Verbindung der Formel V 1-(2-Acetamino-5-pyrimidinyl)-2-aminoethanol und als Verbindung der Formel VI 4-(Dimethylaminomethyl)-phenylaceton ein, läßt sich Verfahren 2c) durch folgendes Reaktionsschema wiedergeben:

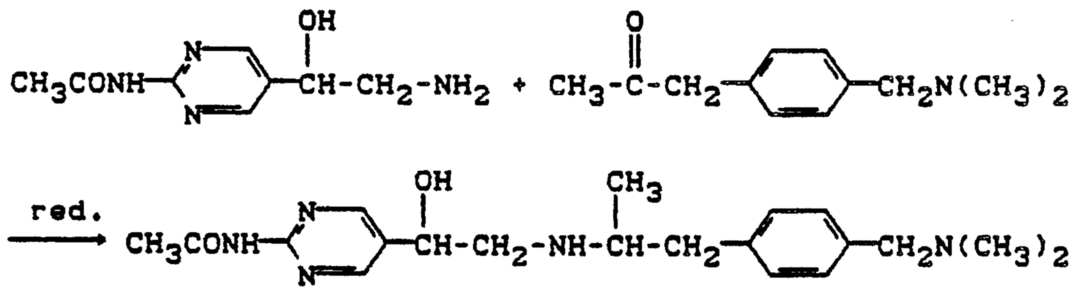


Die Verbindungen der Formel V sind neu. Ihre Herstellung wird weiter unten beschrieben. Die Substituenten $R^1$ bis $R^3$ besitzen bevorzugt die weiter oben bei den Verbindungen der Formel I angegebenen bevorzugten Bedeutungen. Im einzelnen seien folgende Verbindungen der Formel V genannt:

1-(2-Amino-5-pyrimidinyl)-2-amino-ethanol

1-(2-Methylamino-5-pyrimidinyl)-2-aminoethanol

1-(2-Acetamino-5-pyrimidinyl)-2-aminoethanol

Verbindungen der Formel VI sind bekannt (vergl. z. B. EP-OS 70 133). Die Substituenten $R^5$ und $R^6$ besitzen bevorzugt die weiter oben bei den Verbindungen der Formel I angegebenen Bedeutungen.

Im einzelnen seinen folgende Verbindungen der Formel VI genannt:

Aceton,

Methylethylketon,

Methylisobutylketon,

4-Carbomethoxyphenylaceton,

4-(Carbomethoxymethoxy)phenylaceton,

4-(2-Hydroxyethoxy)phenylaceton.

Verfahren 2c) wird durchgeführt, indem man etwa äquimolare Mengen der Verbindungen der Formel V und VI in einem Verdünnungsmittel vorlegt und die Mischung reduziert.

Die Reaktion wird bei Temperaturen von 0° C bis 150° C durchgeführt.

Es wird bevorzugt bei Normaldruck gearbeitet.

Als Verdünnungsmittel dienen alle inerten organischen Lösungsmittel. Hierzu gehören aliphatische und aromatische, gegebenenfalls halogenierte Kohlenwasserstoffe wie Pentan, Hexan, Cyclohexan, Benzol, Toluol, Methylenchlorid, Ethylenchlorid, Chloroform, Chlorbenzol, ferner Ether wie Diethylether, Tetrahydrofuran, Dioxan, weiterhin Nitrile wie Acetonitril, Benzonitril, Amide wie Dimethylformamid. Alkohole wie Methanol, Ethanol.

Als Reduktionsmittel dienen:

$H_2$/Katalysator, als Katalysator sei beispielsweise $PtO_2$ genannt; komplexe Metallhydride wie z. B. $LiAlH_4$, $NaBH_4$, $NaBH_3CN$.

Setzt man bei Verfahren 2d) als Verbindung der Formel VII 2-Acetamino-5-pyrimidinylglyoxal und als Amin der Formel III 3-(4-Methoxyphenyl)-2-propylamin ein, läßt sich Verfahren 2d) durch folgendes Reaktionsschema wiedergeben:

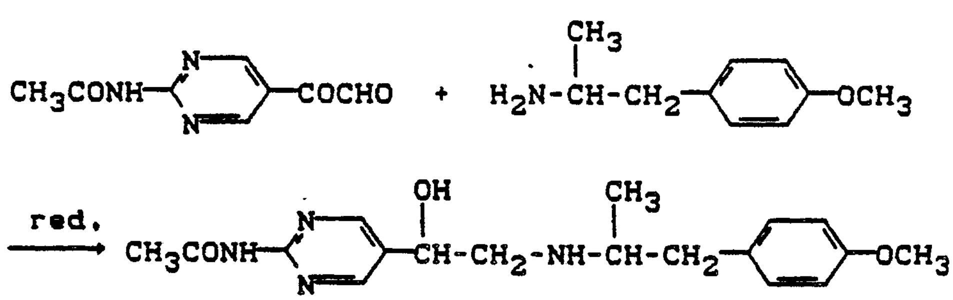


Verbindungen der Formel VII sind neu. Ihre Herstellung wird weiter unten beschrieben. Die Substituenten $R^1$ und $R^2$ besitzen bevorzugt die weiter oben bei den Verbindungen der Formel I angegebenen bevorzugten Bedeutungen. Im einzelnen seien folgende Verbindungen der Formel VII genannt:

2-Amino-5-pyrimidinylglyoxal

2-Methylamino-5-pyrimidinylglyoxal

2-Acetamino-5-pyrimidinylglyoxal

Verfahren 2d) wird durchgeführt, indem man zur Verbindung der Formel VII in einem Verdünnungsmittel etwa die äquivalente Menge des Amins der Formel III zusetzt und anschließend reduziert.

Die Reaktion wird bei Temperaturen von 0 °C bis 100 °C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, außerdem Ester, wie Essigsäuremethylester und -ethylester, ferner Nitrile, wie z. B. Acetonitril und Propionitril, Benzonitril, Glutarsäuredinitril, darüber hinaus Amide, wie z. B. Dimethylformamid, Dimethylacetamid und N-Methylpyrrolidon, sowie Tetramethylensulfon und Hexamethylphosphorsäuretriamid, weiterhin Alkohole wie Methanol, Ethanol, n- und i-Propanol.

Als Reduktionsmittel dienen

$H_2$/Katalysator, als Katalysator seien $PtO_2$ und Pd-Kohle genannt; ferner komplexe Metallhydride wie $LiAlH_4$ und $NaBH_4$.

Wie bereits erwähnt, lassen sich die neuen Verbindungen der Formel II nach den unter 4 angegebenen Verfahren herstellen.

Setzt man bei Verfahren 4a) als Verbindung der Formel VIII 2-Amino-5-acetylpyrimidin und als Halogen Hal Brom ein, läßt sich die Reaktion durch folgendes Schema darstellen.

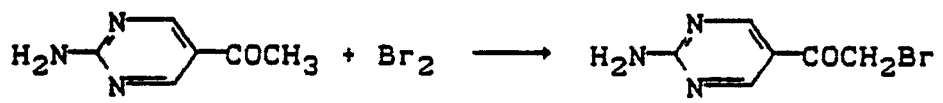

Die Verbindungen der Formel VIII sind neu. Ihre Herstellung wird weiter unten beschrieben. Die Substituenten $R^1$ und $R^2$ besitzen bevorzugt die bei den Verbindungen der Formel I angegebenen Bedeutungen. Im einzelnen seien folgende Verbindungen der Formel VIII genannt:

2-Amino-5-acetylpyrimidin

2-Methylamino-5-acetylpyrimidin

2-Acetamino-5-acetylpyrimidin

Verfahren 4a) wird durchgeführt, indem man zu Verbindung VIII in einem Verdünnungsmittel die äquivalente Menge Halogen, eventuell in einem Verdünnungsmittel gelöst, zugibt.

Die Reaktion wird bei +20 °C bis +150 °C durchgeführt.

Es wird bevorzugt bei Normaldruck gearbeitet.

Als Verdünnungsmittel seien genannt: aliphatische, gegebenenfalls halogenierte Kohlenwasserstoffe wie Pentan, Hexan, Heptan, Cyclohexan, Methylenchlorid, Ethylenchlorid, Chloroform, Tetrachlorkohlenstoff, Alkohole wie Methanol, Ethanol, Ester wie Essigsäureethylester und Mischungen aus diesen Verdünnungsmitteln.

Setzt man bei Verfahren 4b) as Verbindung der Formel VIII 2-Methylamino-4-acetylpyrimidin und als Verbindung der Formel $CuHal_2$ Kupfer(II)-bromid ein, läßt sich die Reaktion durch folgendes Schema darstellen:

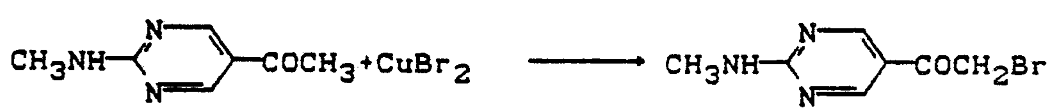

Verfahren 4b) wird durchgeführt, indem man äquivalente Mengen der Verbindung der Formel VIII und der Verbindung $CuHal_2$ im Verdünnungsmittel 1 - 24 h, bevorzugt 6 - 12 h, unter Rückfluß erhitzt.

Die übrigen Reaktionsparameter sowie die Verdünnungsmittel sind bei Verfahren 4a) beschrieben.

Wie bereits erwähnt, lassen sich die neuen Verbindungen der Formel IV nach dem unter 6) angegebenen Verfahren herstellen.

Setzt man bei Verfahren 6) als Verbindung der Formel II (2-Acetamino-5-pyrimidinyl)-chlormethylketon ein, läßt sich das Verfahren durch folgendes Reaktionsschema darstellen:

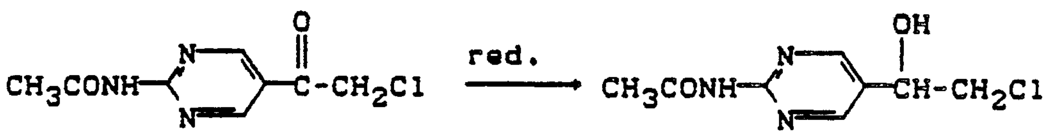


Die Substituenten R[1], R[2] und Hal in den Verbindungen der Formel II besitzen die weiter oben angegebenen bevorzugten Bedeutungen.

Als Reduktionsmittel zur Durchführung des Verfahrens seien genannt:
$H_2$/Katalysator, als Katalysatoren seien genannt: $PtO_2$, Pd/Kohle; komplexe Metallhydride, wie z. B. $LiAlH_4$, $NaBH_4$, $NaBH_3CN$. Bevorzugt werden $NaBH_4$ und $NaBH_3CN$ eingesetzt.

Das Verfahren wird durchgeführt, indem die Verbindung II in einem Verdünnungsmittel mit dem Reduktionsmittel umsetzt.

Die Reaktion wird bei Temperaturen von -20° C bis +100° C durchgeführt.

Es wird bevorzugt bei Normaldruck gearbeitet.

Als Verdünnungsmittel dienen alle inerten organischen Lösungsmittel. Hierzu zählen insbesondere gegebenenfalls halogenierte aliphatische und aromatische Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril, Benzonitril; Alkohole wie Methanol, Ethanol, n- und i-Propanol. Bevorzugt werden Alkohole eingesetzt.

Wie bereits erwähnt, lassen sich die neuen Verbindungen der Formel V nach den unter 8) angegebenen Verfahren herstellen.

Setzt man bei Verfahren 8a) als Verbindung der Formel IX 1-(2-Methylamino-5-pyrimidinyl)-2-nitroethanol ein, läßt sich das Verfahren durch folgendes Schema wiedergeben:

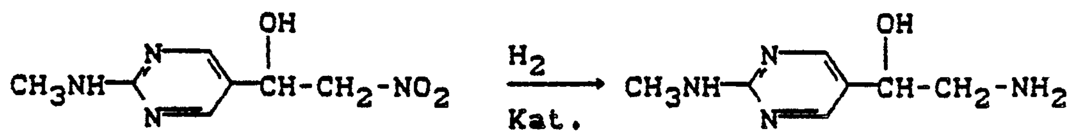


Verbindungen der Formel IX sind neu. Ihre Herstellung wird weiter unten beschrieben. Die Substituenten R[1] bis R[3] besitzen bevorzugt die bei den Verbindungen der Formel I angegebenen Bedeutungen. Im einzelnen seien folgende Verbindungen der Formel IX genannt:
1-(2-Amino-5-pyrimidinyl)-2-nitroethanol
1-(2-Methylamino-5-pyrimidinyl)-2-nitroethanol
1-(2-Acetamino-5-pyrimidinyl)-2-nitroethanol

Als Reduktionsmittel für das Verfahren dient Wasserstoff/Katalysator. Als Katalysatoren seien beispielhaft genannt: Raney-Nickel, $PtO_2$, Pd/Kohle.

Das Verfahren wird durchgeführt, indem man Verbindung IX in einem Verdünnungsmittel katalytisch hydriert.

Die Reaktion wird bei Temperaturen von +20° C bis +150° C durchgeführt.

Es wird unter Normaldruck oder erhöhtem Druck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Benzol, Toluol; Ether wie Diethylether, Tetrahydrofuran; Alkohole wie Methanol, Ethanol.

Setzt man bei Verfahren 8b) als Verbindung der Formel X (2-Amino-5-pyrimidinyl)-cyanhydrin ein, läßt sich das Verfahren durch folgendes Reaktionsschema darstellen:

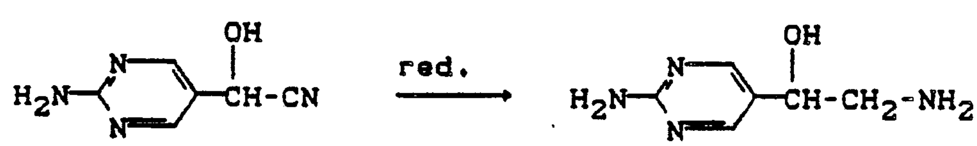


Verbindungen der Formel X sind neu. Ihre Herstellung wird weiter unten beschrieben. Die Substituenten R[1] und R[2] besitzen bevorzugt die bei den Verbindungen der Formel I angegebenen Bedeutungen. Im einzelnen seien folgende Verbindungen der Formel X genannt:
(2-Amino-5-pyrimidinyl)-cyanhydrin

(2-Methylamino-5-pyrimidinyl)-cyanhydrin
(2-Acetamino-5-pyrimidinyl)-cyanhydrin

Das Verfahren wird durchgeführt, indem man die Verbindung X in einem Verdünnungsmittel reduziert.

Die Reaktion wird bei Temperaturen von 0 °C bis 150 °C durchgeführt.

Es wird bei Normaldruck oder bei erhöhtem Druck gearbeitet.

Als Verdünnungsmittel dienen, abhängig vom Reduktionsmittel, Wasser oder organische Lösungsmittel oder Mischungen hiervon. Zu den organischen Lösungsmitteln gehören aliphatische und aromatische, gegebenenfalls halo genierte Kohlenwasserstoffe, wie Pentan, Hexan, Cyclohexan, Benzol, Toluol, Methylenchlorid, Ethylenchlorid, Chloroform, Chlorbenzol, ferner Ether wie Diethylether, Tetrahydrofuran, Dioxan, weiterhin Nitrile wie Acetonitril, Benzonitril, Amide wie Dimethylformamid, Alkohole wie Methanol, Ethanol.

Als Reduktionsmittel dienen:
$H_2$/Katalysator; als Katalysator sei beispielsweise $PtO_2$ genannt; Alkali- und Erdalkaliamalgame wie z. B. Natriumamalgam; unedle Metalle in Gegenwart von Salzsäure wie z. B. Zink/Salzsäure; komplexe Metallhydride wie z. B. $LiAlH_4$; Borane wie z. B. Diboran.

Wie bereits erwähnt, lassen sich die neuen Verbindungen der Formel VII nach dem unter 10 angegebenen Verfahren herstellen.

Setzt man bei Verfahren 10) als Verbindung der Formel II (2-Acetamino-5-pyrimidinyl)-chlormethylketon ein, läßt sich das Verfahren durch das folgende Schema darstellen:

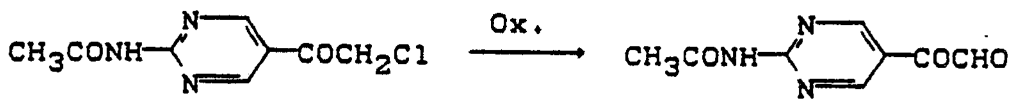


Als Halogenmethylketone der Formel II werden bevorzugt wie weiter oben genannten Verbindungen eingesetzt.

Das Verfahren 10) wird durchgeführt, indem man die Verbindungen der Formel II, gegebenenfalls in Gegenwart eines Verdünnungsmittels, oxidiert.

Die Reaktion wird bei Temperaturen von +20 °C bis +100 °C durchgeführt.

Es wird bevorzugt bei Normaldruck gearbeitet.

Als Oxidationsmittel wird bevorzugt Dimethylsulfoxid verwendet [N. Kornblum et. al., JACS 79, 6562 (1957)].

Wird die Umsetzung in Gegenwart eines Verdünnungsmittels durchgeführt, können alle inerten organischen Lösungsmittel verwendet werden. Hierzu gehören insbesondere aliphatische und aromatische, gegebenenfalls halogenierte Kohlenwasserstoffe wie Pentan, Hexan, Cyclohexan, Benzol, Toluol, Methylenchlorid, Chloroform, Chlorbenzol; Ether wie Diethylether, Tetrahydrofuran; Nitrile wie Acetonitril, Benzonitril. Bevorzugt wird in Dimethylsulfoxid ohne ein weiteres Lösungsmittel gearbeitet.

Wie bereits erwähnt, lassen sich die neuen Verbindungen der Formel VIII nach dem unter 12) beschriebenen Verfahren herstellen.

Setzt man bei Verfahren 12) als Verbindung der Formel XI 2-Methylsulfonyl-5-acetylpyrimidin und als Amin der Formel XII Dimethylamin ein, läßt sich das Verfahren durch folgendes Schema darstellen:

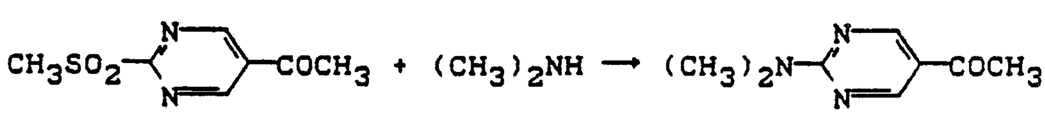

Verbindungen der Formel XI sind bekannt [Acta Chem. Scand. B73 (1985), 235].

Bevorzugt werden folgende Verbindungen der Formel XI eingesetzt:
2-Methylsulfonyl-5-acetylpyrimidin
2-Ethylsulfonyl-5-acetylpyrimidin
2-Tosyl-5-acetylpyrimidin
2-Brom-5-acetylpyrimidin
2-Chlor-5-acetylpyrimidin

Amine der Formel XII sind bekannt. Die Substituenten $R^1$ und $R^2$ besitzen bevorzugt die bei den Verbindungen der Formel I angegebenen Bedeutungen. Im einzelnen seien folgende Amine der Formel XII genannt:
Ammoniak
Methylamin

Dimethylamin
Pyrrolidin
Piperidin
Morpholin

Verfahren 12) wird durchgeführt, indem man die Verbindung der Formel XI mit der 2- bis 5-fachen, bevorzugt 2-bis 3-fachen molaren Menge Amin der Formel XII, gegebenenfalls in einem Verdünnungsmittel umsetzt.

Als Verdünnungsmittel können alle inerten organischen Lösungsmittel verwendet werden. Hierzu gehören insbesondere gegebenenfalls halogenierte aliphatische oder aromatische Kohlenwasserstoffe wie Pentan, Hexan, Heptan, Cyclohexan, Benzol, Toluol, Dichlormethan, Chloroform, Dichlorethan, Chlorbenzol, weiterhin Ether wie Diethylether, Tetrahydrofuran oder Dioxan, ebenso Amide wie Dimethylformamid, ebenso Wasser.

Die Reaktion wird bei Temperaturen von -20 °C bis +250 °C durchgeführt.

Es wird bei Normaldruck oder unter erhöhtem Druck gearbeitet.

Wie bereits erwähnt, lassen sich die neuen Verbindungen der Formel IX nach dem unter 14) beschriebenen Verfahren herstellen.

Setzt man bei Verfahren 14) als Verbindung der Formel XIII 2-Acetamino-5-pyrimidinaldehyd ein, läßt sich das Verfahren durch folgendes Schema wiedergeben:

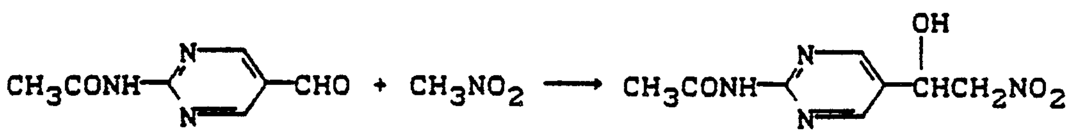

Verbindungen der Formel XIII sind neu. Ihre Herstellung wird weiter unten beschrieben. Die Substituenten $R^1$ und $R^2$ besitzen bevorzugt die bei den Verbindungen der Formel I angegebenen Bedeutungen. Im einzelnen seien folgende Verbindungen der Formel XIII genannt:
2-Aminopyrimidin-5-aldehyd
2-Methylaminopyrimidin-5-aldehyd
2-Acetaminopyrimidin-5-aldehyd

Verfahren 14) wird durchgeführt, indem man äquivalente Mengen der Verbindung XIII und Nitromethan in einem Verdünnungsmittel in Gegenwart einer Base umsetzt.

Die Reaktion wird bei Temperaturen von -20 °C bis +50 °C durchgeführt.

Es wird bevorzugt bei Normaldruck gearbeitet.

Als Verdünnungsmittel dienen alle inerten organischen Lösungsmittel. Hierzu gehören insbesondere Ether wie Diethylether, Tetrahydrofuran; Alkohole wie Methanol, Ethanol.

Als Basen werden verwendet: Alkali- und Erdalkalihydroxide wie Natrium- und Kaliumhydroxid, -alkoholate wie Natrium- und Kaliummethylat, Natrium- und Kaliumethylat.

Wie bereits erwähnt, lassen sich die neuen Verbindungen der Formel X nach dem unter 16) beschriebenen Verfahren herstellen.

Setzt man bei Verfahren 16) als Verbindung der Formel XIII 2-Methylaminopyrimidin-5-aldehyd ein, läßt sich das Verfahren durch folgendes Schema darstellen:

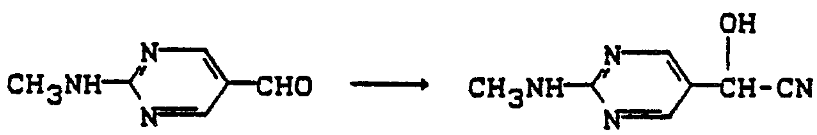

Als Verbindungen der Formel XIII werden bevorzugt die oben genannten Verbindungen eingesetzt.

Das Verfahren wird durchgeführt, indem man in literaturbekannter Weise die Aldehyde der Formel XIII bzw. deren Hydrogensulfit-Additionsprodukte mit Cyanwasserstoff, dessen Salzen oder mit Ketoncyanhydrinen umsetzt (Houben-Weyl, Bd. VIII, S. 274 ff.).

Wie bereits erwähnt, lassen sich die neuen Verbindungen der Formel XIII nach dem unter 18) beschriebenen Verfahren herstellen.

Setzt man bei Verfahren 18) als Verbindung der Formel XIV (2-Methylamino-5-pyrimidinyl)-methanol ein, läßt sich das Verfahren durch folgendes Reaktionsschema wiedergeben:

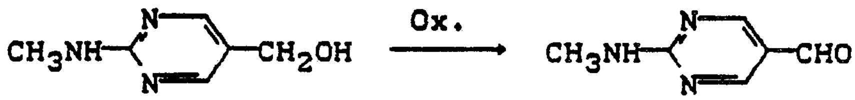


Verbindungen der Formel XIV sind neu. Ihre Herstellung wird weiter unten beschrieben. Die Substituenten $R^1$ und $R^2$ besitzen bevorzugt die bei den Verbindungen der Formel I angegebenen Bedeutungen. Im einzelnen seien folgende Verbindungen der Formel XIV genannt:
(2-Amino-5-pyrimidinyl)-methanol
(2-Methylamino-5-pyrimidinyl)-methanol
(2-Acetamino-5-pyrimidinyl)-methanol

Als Oxidationsmittel wird bevorzugt Mangandioxid eingesetzt.

Verfahren 18) wird durchgeführt, indem man den Alkohol der Formel XIV mit überschüssigem Mangandioxid umsetzt.

Die Reaktion wird bei Temperaturen von +20° C bis +150° C durchgeführt, es wird bevorzugt bei Normaldruck gearbeitet.

Als Verdünnungsmittel werden inerte organische Lösungsmittel verwendet. Hierzu zählen insbesondere aliphatische und aromatische, gegebenenfalls halogenierte Kohlenwasserstoffe wie Pentan, Hexan, Cyclohexan, Benzol, Toluol, Methylenchlorid, Chloroform, Ether wie Diethylether und Tetrahydrofuran, Ketone wie Aceton und Methylisobutylketon.

Wie bereits erwähnt, lassen sich die neuen Verbindungen der Formel XIV nach dem unter 20) beschriebenen Verfahren herstellen.

Setzt man bei Verfahren 20) als Verbindung der Formel XV 2-Methylaminopyrimidin-5-carbonsäuremethylester ein, läßt sich das Verfahren durch folgendes Schema wiedergeben:

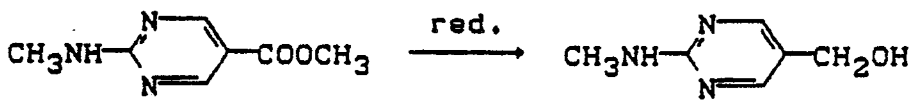


Verbindungen der Formel XV sind bekannt [JACS 64 (1942), 794]. Die Substituenten $R^1$ und $R^2$ besitzen bevorzugt die bei dem Verbindungen der Formel I angegebenen Bedeutungen, $R^{11}$ steht für Wasserstoff, Methyl, Ethyl. Im einzelnen seien folgende Verbindungen der Formel XV genannt:
2-Aminopyrimidin-5-carbonsäure
2-Aminopyrimidin-5-carbonsäuremethylester
2-Methylaminopyrimidin-5-carbonsäure
2-Methylaminopyrimidin-5-carbonsäureethylester
2-Acetaminopyrimidin-5-carbonsäure

Als Reduktionsmittel werden eingesetzt:
für Verbindungen der Formel XV, in denen $R^{11}$ für Wasserstoff steht, Borane wie z. B. Diboran, komplexe Metallhydride wie z. B. $LiAlH_4$;
für Verbindungen der Formel XV, in denen $R^{11}$ für Methyl oder Ethyl steht, komplexe Metallhydride wie z. B. $LiAlH_4$.

Das Verfahren wird durchgeführt, indem man die Verbindungen der Formel XV in einem Verdünnungsmittel mit der 1 - 4fachen molaren Menge Reduktionsmittel umsetzt. Die Reaktion wird bei Temperaturen von -50° C bis +100° C durchgeführt, es wird bevorzugt bei Normaldruck gearbeitet. Als Verdünnungsmittel werden inerte organische Lösungsmittel verwendet, vorzugsweise Ether wie Diethylether und Tetrahydrofuran.

Die Wirkstoffe eignen sich bei günstiger Warmblütertoxizität als Mittel zur Leistungsförderung bei Zucht- und Nutztieren. Sie dienen dabei zur Förderung und Beschleunigung des Wachstums, der Milch- und Wollproduktion sowie zur Verbesserung der Futterverwertung, der Fleischqualität und zur Verschiebung des Fleisch-Fett-Verhältnisses zugunsten von Fleisch.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z. B. Nerze, Chinchilla, Waschbär; Vögel wie z.B. Hühner, Gänse, Puten, Enten; Süß- und Salzwasserfische wie z.B. Forellen, Karpfen, Aale.

Die Wirkstoffe werden unabhängig vom Geschlecht der Tiere während allen Wachstums- und Leistungsphasen der Tiere eingesetzt. Bevorzugt werden die Wirkstoffe während der intensiven Wachstums- und Leistungsphase eingesetzt. Die intensive Wachstums- und Leistungsphase dauert je nach Tierart von einem Monat bis zu 10 Jahren. Besonders wertvoll erweisen sich die Wirkstoffe bei Aufzucht und Haltung

von Jung- und Masttieren.

Die Anwendung der Wirkstoffe erfolgt direkt oder in Form von für Tiere geeigneten Zubereitungen enteral oder parenteral. Die enterale Anwendung der Wirkstoffe geschieht z.B. oral in Form von Pulvern, Tabletten, Kapseln, Pasten, Tränken, Granulaten, Boli, über oral applizierbare Lösungen, Emulsionen oder Suspensionen sowie über das Futter oder über das Trinkwasser. Die parenterale Anwendung geschieht z.B. in Form der Injektion (intramuskulär, subcutan, intravenös) oder durch Implantate.

Besonders hervorgehoben seien Zubereitungen zur Verabreichung über das Futter oder das Trinkwasser. Dabei können die Wirkstoffe dem Futter direkt oder in Form von Prämixen oder Futterkonzentraten zugesetzt werden.

Zum Futter zählen Einzelfuttermittel pflanzlicher Herkunft wie Heu, Rüben, Getreide und Getreidenebenprodukte, Melasse, Silage, Einzelfuttermittel tierischer Herkunft wie Fleisch, Fette, Milchprodukte, Knochenmehl, Fischprodukte, die Einzelfuttermittel wie Vitamine, Proteine, Zucker, Stärke, Mehle, Aminosäuren z.B. DL-Methionin, Salze wie Kalk und Kochsalz. Zum Futter zählen auch Ergänzungs-, Fertig-und Mischfuttermittel. Diese enthalten Einzelfuttermittel in einer Zusammensetzung, die eine ausgewogene Ernährung hinsichtlich der Energie- und Proteinversorgung sowie der Versorgung mit Vitaminen, Mineralsalzen und Spurenelementen sicherstellt.

Prämixe und Futterkonzentrate sind Mischungen des Wirk stoffs mit Trägerstoffen und gegebenenfalls weiteren Hilfsstoffen. Zu den Trägerstoffen zählen alle Einzelfuttermittel oder Gemische derselben.

Die Wirkstoffe können in den Zubereitungen allein oder in Mischung mit anderen leistungsfördernden Wirkstoffen, mineralischen Futtermitteln, Spurenelement-Verbindungen, Vitaminen, stickstoffhaltigen Nicht-Protein-Verbindungen, Farbstoffen, Antioxidantien, Aromastoffen, Emulgatoren, Fließhilfsstoffen, Konservierungs- und Preßhilfsstoffen vorliegen.

Andere leistungsfördernde Wirkstoffe sind z.B. Antibiotika wie Tylosin und Virginiamycin.

Mineralische Futtermittel sind z.B. Dicalciumphosphat, Magnesiumoxid, Natriumchlorid.

Spurenelement-Verbindungen sind z.B. Eisenfumarat, Natriumiodid, Kobaltchlorid, Kupfersulfat, Zinkoxid, Selenverbindungen.

Vitamine sind z.B. Vitamin A, Vitamin $D_3$, Vitamin E.

Stickstoffhaltige Nicht-Protein-Verbindungen sind z.B. Biuret, Harnstoff.

Farbstoffe sind z.B. Carotinoide wie Canthaxanthin, Zeaxanthin, Capsanthin oder Farbstoffe, die zur Färbung von Lebensmitteln zugelassen sind.

Antioxidantien sind z.B. Ethoxyquin, Butylhydroxy-toluol, Ascorbinsäure.

Aromastoffe sind z.B. Vanillin.

Emulgatoren sind z.B. Ester der Milchsäure, Lecithin. Fließhilfsstoffe sind z.B. Natriumstearat, Calciumstearat, Kieselsäuren, Bentonite, Ligninsulfonate.

Konservierungsstoffe sind z.B. Propionsäure, Calciumpropionat, Sorbinsäure, Ascorbinsäure. Preßhilfsstoffe sind z.B. Ligninsulfonate, Celluloseether.

Die Konzentration der Wirkstoffe im Futter beträgt normalerweise etwa 0,001-500 ppm, bevorzugt 0,1-50 ppm.

Die Konzentration der Wirkstoffe in den Prämixen oder Futterkonzentraten beträgt ca. 0,5 bis 50 Gewichtsprozent, bevorzugt 1 bis 20 Gewichtsprozent.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstums- und Leistungsphase temporär oder kontinuierlich verabreicht werden. Bei kontinuierlicher Verabreichung kann die Anwendung ein-oder mehrmals täglich in regelmäßigen oder unregelmäßigen Abständen erfolgen.

Beispiel für die Zusammensetzung eines Kükenaufzuchtfutters, das erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g iodiertes Kochsalz, 7,5 g Vitamin-Mineral-Mischung der unten angegebenen Zusammensetzung und 2.5 g Wirkstoff-Prämix der unten angegebenen Zusammensetzung ergeben nach sorgfältigem Mischen 1 kg Futter.

In einem kg Vitamin-Mineral-Mischung sind enthalten: 600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $MnSO_4 \times H_2O$, 140 mg $ZnSO_4 \times 7\ H_2O$, 100 mg $FeSO_4 \times 7\ H_2O$ und 20 mg $CuSO_4 \times 5\ H_2O$ in Getreidemehl als Trägerstoff.

1 kg Wirkstoff-Prämix enthalten 100 g Wirkstoff, 900 g Weizenmehl.

Beispiel für die Zusammensetzung eines Schweineaufzuchtfutters, das erfindungsgemäßen Wirkstoff enthält:

630 g Futtergetreideschrot (zusammengesetzt aus 200 g Mais, 150 g Gerste-, 150 g Hafer- und 130 g Weizenschrot), 80 g Fischmehl, 60 g Sojaschrot, 60 g Tapiokamehl, 38 g Bierhefe, 50 g Vitamin-Mineral-Mischung (Zusammensetzung wie beim Kükenfutter) 30 g Leinkuchenmehl, 30 g Maiskleberfutter, 10 g Sojaöl, 10 g Zuckerrohrmelasse und 2 g Wirkstoff-Prämix ergeben nach sorgfältigem Mischen 1 kg Futter. 1 kg Wirkstoff-Prämix enthält 200 g Wirkstoff, 20 g Pflanzenöl, 780 g Calciumcarbonatpulver.

Beispiel für die Zusammensetzung eines Futters für Rinder, das den erfindungsgemäßen Wirkstoff enthält:

69,95 % Futtergetreideschrot, 10 % gemahlene Maiskolben, 8 % Sojabohnenmehl, 5 % Luzernemehl, 5 % Melasse, 0,6 % Harnstoff, 0,5 % Calciumphosphat, 0,5 % Calciumcarbonat, 0,3 % Kochsalz, 0,15 % Vitamin-Mineral-Mischung und 0,2 % Wirkstoff-Prämix der beim Schweineaufzuchtfutter angegebenen Zusammensetzung. Die Vitamin-Mineral-Mischung enthält pro kg 70.000 i.E. Vitamin A, 70.000 i:E. Vitamin $D_3$, 100 mg Vitamin E, 50 mg $MnSO_4 \times H_2O$, 30 mg $ZnSO_4 \times 7H_2O$ in Getreidemehl als Trägerstoff.

Der Wirkstoff-Prämix wird der Vitamin-Mineral-Mischung in der erforderlichen Menge beigemischt und diese anschließend sorgfältig mit den übrigen Bestandteilen vermischt.

## Beispiel

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist, gefüttert. Eine Kontrollgruppe erhält Futter ohne Wirkstoff. Die Versuchsgruppen werden so zusammengestellt, daß das durchschnittliche Körpergewicht sowie die Streuung in den Körpergewichten der Ratten in jeder Versuchsgruppe gleich ist, so daß eine Vergleichbarkeit der Versuchsgruppen untereinander gewährleistet ist.

Die Tiere werden vor Versuchsbeginn 2 Tage an die neuen Haltungsbedingungen adaptiert, während dieser Zeit wird Futter ohne Wirkstoffzusatz gegeben. Danach erhalten die Tiere 13 Tage lang Futter, das Wirkstoff enthält. Es wird die relative Gewichtszunahme im Verhältnis zur unbehandelten Kontrolle bestimmt.

Es werden die aus der Tabelle ersichtlichen Ergebnisse erhalten:

Tabelle

| Ratten-Fütterungsversuch | | |
|---|---|---|
| Wirkstoff Bsp.-Nr. | Wirkstoffkonzentration ppm | relative Gewichtszunahme |
| 1 | 100 | 30 % |

Herstellungsbeispiele:

1. 1-(2-Amino-5-pyrimidinyl)-2-(tert.-butylamino)-ethanol

In eine Lösung von 6 g (0,08 mol) tert.-Butylamin in 120 ml Methanol werden portionsweise 1,28 g (5,9 mmol) (2-Amino-5-pyrimidinyl)-brommethylketon eingetragen. Man rührt 30 min bei 40 °C nach, kühlt auf 0 °C und gibt 340 mg (8,9 mmol) $NaBH_4$ zu. Man rührt 30 min nach und dampft dann ein. Der Rückstand wird zwischen Essigester und Wasser verteilt, die organische Phase wird abgetrennt, über $Na_2SO_4$ getrocknet und eingedampft.

Ausbeute: 920 mg (74 % d. Th.), Fp. 110 - 111 °C

Analog werden erhalten:

2. 1-(2-Amino-5-pyrimidinyl)-2-((1,1-dimethyl-2-hydroxyethyl)amino)-ethanol, Fp. 75 °C

3. 1-(2-Amino-5-pyrimidinyl)-2-(2-cyclohexylethylamino)-ethanol, Fp. 164-165 °C

Herstellung der Ausgangsverbindungen der Formel VIII:

a) 2-Amino-5-acetylpyrimidin

In eine Mischung von 50 ml konz. wässrigen Ammoniak und 50 ml Tetrahydrofuran werden portionsweise 7 g (33 mmol) 2-Ethylsulfonyl-5-acetylpyrimidin eingetragen. Man rührt 30 min bei Raumtemperatur nach und dampft das Tetrahydrofuran ab. Das ausgefallene Produkt wird abgesaugt, das Filtrat noch dreimal mit je 25 ml Essigester extrahiert. Die vereinigten organischen Phasen werden über $Na_2SO_4$ getrocknet und eingedampft.

Man erhält 3,9 g (87 % d. Th.), Fp. 172 - 173 °C

Herstellung der Ausgangsverbindungen der Formel II

b) (2-Amino-5-pyrimidinyl)-brommethylketon

Zur Lösung von 2 g (15 mmol) 2-Amino-5-acetylpyrimidin in 100 ml Eisessig werden zunächst 2,7 g 48 %ige wässrige HBr ( ≙ 16 mmol) HBr, dann 2,4 g (15 mmol) Brom gegeben. Man rührt 90 min bei 40 °C nach und saugt das ausgefallene Hydrobromid des Produkts ab. Dieses wird mit überschüssiger, gesättigter, wässriger $NaHCO_3$-Lösung verrührt, die freie Base wird abfiltriert.

Ausbeute: 2,6 g (81 % d. Th.), Fp. > 230 °C $^1$H-NMR (DMSO-$d_6$,δ) : 4,75 (s,2H); 7,8 (s (breit), 2H); 8,8 (s,2H).

## Ansprüche

1. Pyrimidinderivate der Formel I

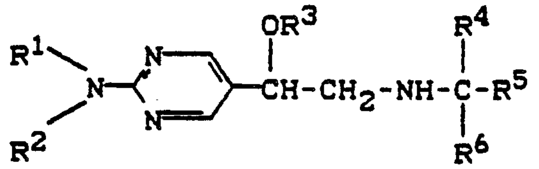

(I)

in welcher

$R^1$ für Wasserstoff, Alkyl, Acyl steht,

$R^2$ für Wasserstoff, Alkyl steht,

$R^1$ und $R^2$ können gemeinsam mit dem Stickstoffatom, an das sie gebunden sind, einen gesättigten oder ungesättigten 5-Ring oder 6-Ring bilden, der noch weitere Heteroatome enthalten kann,

$R^3$ für Wasserstoff, Alkyl, Acyl steht,

$R^4$ für Wasserstoff, Alkyl, Halogenalkyl steht,

$R^5$ für Alkyl, Halogenalkyl steht,
$R^6$ für Alkyl oder Cycloalkyl steht, die gegebenenfalls substituiert sind durch Halogen, Cycloalkyl, Hydroxy, Alkoxy, Halogenalkoxy, Aryl, das seinerseits substituiert sein kann durch Alkyl, Halogen, Hydroxy, Alkoxy, Alkylendioxy, CN oder die Reste -$COR^7$, -O-Alkylen-$COR^7$, Alkylen-$R^8$ oder O-Alkylen-$R^8$,
$R^7$ für Hydroxy, Alkoxy oder -$NR^9R^{10}$ steht,
$R^8$ für Hydroxy, Alkoxy oder -$NR^9R^{10}$ steht,
$R^9$ für Wasserstoff, Alkyl steht,
$R^{10}$ für Wasserstoff, Alkyl steht,
sowie deren tautomere Formen für den Fall, daß mindestens einer der Reste $R^1$ oder $R^2$ für Wasserstoff steht, und physiologisch verträglichen Salze.

2. Verfahren zur Herstellung der neuen Pyrimidinderivate der Formel I

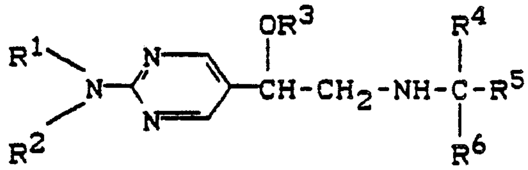

(I)

in welcher
$R^1$ für Wasserstoff, Alkyl, Acyl steht,
$R^2$ für Waserstoff, Alkyl steht,
$R^1$ und $R^2$ können gemeinsam mit dem Stickstoffatom, an das sie gebunden sind, einen gesättigten oder ungesättigten 5-Ring oder 6-Ring bilden, der noch weitere Heteroatome enthalten kann,
$R^3$ für Wasserstoff, Alkyl, Acyl steht,
$R^4$ für Wasserstoff, Alkyl, Halogenalkyl steht,
$R^5$ für Alkyl, Halogenalkyl steht,
$R^6$ für Alkyl oder Cycloalkyl steht, die gegebenenfalls substituiert sind durch Halogen, Cycloalkyl, Hydroxy, Alkoxy, Halogenalkoxy, Aryl, das seinerseits substituiert sein kann durch Alkyl, Halogen, Hydroxy, Alkoxy, Alkylendioxy, CN oder die Reste -$COR^7$, -O-Alkylen-$COR^7$, Alkylen-$R^8$ oder O-Alkylen-$R^8$,
$R^7$ für Hydroxy, Alkoxy oder -$NR^9R^{10}$ steht,
$R^8$ für Hydroxy, Alkoxy oder -$NR^9R^{10}$ steht,
$R^9$ für Wasserstoff, Alkyl steht,
$R^{10}$ für Wasserstoff, Alkyl steht,
sowie deren Tautomere und physiologisch verträglichen Salze,
dadurch gekennzeichnet, daß man

a) Halogenmethylketone der Formel II

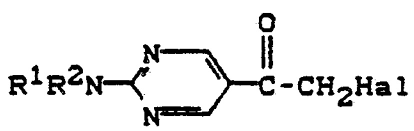

(II)

in welcher
$R^1$ und $R^2$ die oben angegebene Bedeutung haben und
Hal für Halogen steht,
mit Aminen der Formel III

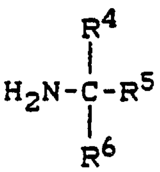

(III)

in welcher
$R^4$ bis $R^6$ die oben angegebene Bedeutung haben,

umsetzt und anschließend die Carbonylgruppe reduziert oder

b) β-Halogenethylverbindungen der Formel IV

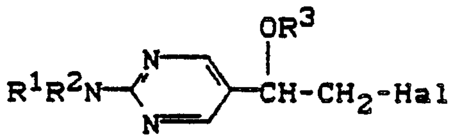

(IV)

in welcher

$R^1$ bis $R^3$ die oben angegebene Bedeutung haben und

Hal für Halogen steht,

mit Aminen der Formel III

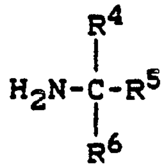

(III)

in welcher

$R^4$ bis $R^6$ die oben angegebene Bedeutung haben,

umsetzt oder

c) für den Fall, daß in Formel I $R^4$ für Wasserstoff steht, indem man Verbindungen der Formel V

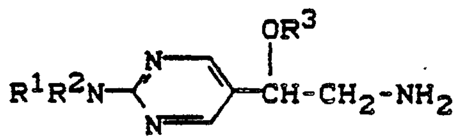

(V)

in welcher

$R^1$ bis $R^3$ die oben angegebene Bedeutung haben

mit Verbindungen der Formel VI

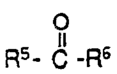

(VI)

in welcher

$R^5$ und $R^6$ die oben angegebene Bedeutung haben,

unter reduzierenden Bedingungen umsetzt oder

d) Verbindungen der Formel VII

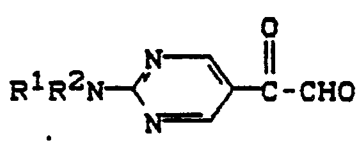

(VII)

in welcher

$R^1$ und $R^2$ die oben angegebene Bedeutung haben,

mit Aminen der Formel III

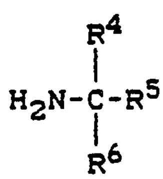

(III)

in welcher
$R^4$ bis $R^6$ die oben angegebene Bedeutung haben,
unter reduzierenden Bedingungen umsetzt.

3. Neue Verbindungen der Formel II

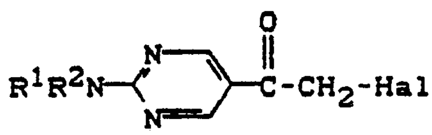

(II)

in welcher
$R^1$ für Wasserstoff, Alkyl, Acyl,
$R^2$ für Wasserstoff, Alkyl,
Hal für Halogen steht.

4. Verfahren zur Herstellung der neuen Verbindungen der Formel II gemäß Anspruch 3, dadurch gekennzeichnet, daß man Acetylverbindungen der Formel VIII

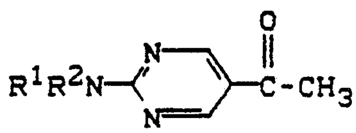

(VIII)

in welcher
$R^1$ für Wasserstoff, Alkyl, Acyl,
$R^2$ für Wasserstoff, Alkyl steht,
a) mit elementarem Halogen,
b) mit Kupferhalogeniden der Formel $CuHal_2$ umsetzt.

5. Neue Verbindungen der Formel IV

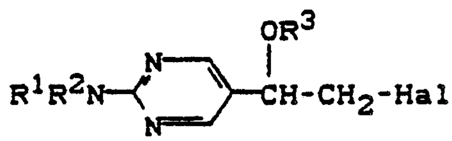

(IV)

in welcher
$R^1$ für Wasserstoff, Alkyl, Acyl steht,
$R^2$ für Wasserstoff, Alkyl steht,
$R^3$ für Wasserstoff, Alkyl, Acyl steht.

6. Verfahren zur Herstellung der neuen Verbindungen der Formel IV gemäß Anspruch 5, dadurch gekennzeichnet, daß man Verbindungen der Formel II

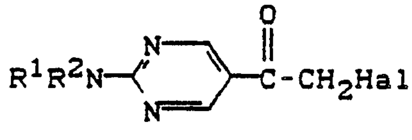

(II)

in welcher
$R^1$, $R^2$ und Hal die bei Anspruch 5 angegebenen Bedeutungen haben,
reduziert und gegebenenfalls alkyliert oder acyliert.

7. Neue Verbindungen der Formel V

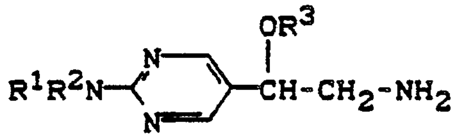

(V)

in welcher
$R^1$ für Wasserstoff, Alkyl, Acyl steht,
$R^2$ für Wasserstoff, Alkyl steht,
$R^3$ für Wasserstoff, Alkyl, Acyl steht.

8. Verfahren zur Herstellung der neuen Verbindungen der Formel V gemäß Anspruch 7, dadurch gekennzeichnet, daß man

a) Verbindungen der Formel IX

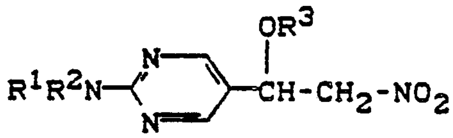

(IX)

in welcher
$R^1$ bis $R^3$ die in Anspruch 7 angegebene Bedeutung haben,
katalytisch reduziert,

b) für den Fall, daß $R^3$ für Wasserstoff steht, indem man Verbindungen der Formel X

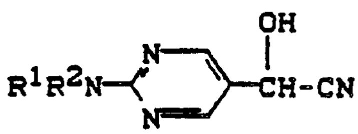

(X)

in welcher
$R^1$ und $R^2$ die in Anspruch 7 angegebene Bedeutung haben,
reduziert.

9. Neue Verbindungen der Formel VII

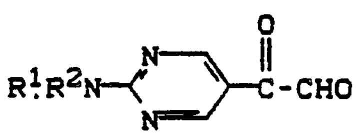

(VII)

in welcher
$R^1$ für Wasserstoff, Alkyl, Acyl steht,
$R^2$ für Wasserstoff, Alkyl steht.

10. Verfahren zur Herstellung der Verbindungen der Formel VII gemäß Anspruch 9, dadurch gekennzeichnet, daß man Verbindungen der Formel II

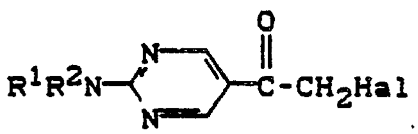

(II)

in welcher
$R^1$ und $R^2$ die in Anspruch 9 angegebene Bedeutung haben und
Hal für Halogen steht,
oxidiert.

11. Neue Verbindungen der Formel VIII

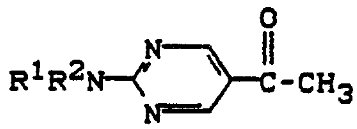

(VIII)

in welcher
$R^1$ für Wasserstoff, Alkyl, Acyl steht,
$R^2$ für Wasserstoff, Alkyl steht.

12. Verfahren zur Herstellung der Verbindungen der Formel VIII gemäß Anspruch 11, dadurch gekennzeichnet, daß man Verbindungen der Formel XI

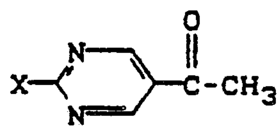

(XI)

in welcher
X für Halogen, $-SO_2$-Alkyl, $-SO_2$-Aryl steht,
mit Aminen der Formel XII
$HNR^1R^2$ (XII)
in welcher
$R^1$ und $R^2$ die in Anspruch 11 angegebene Bedeutung haben,
umsetzt.

13. Neue Verbindungen der Formel IX

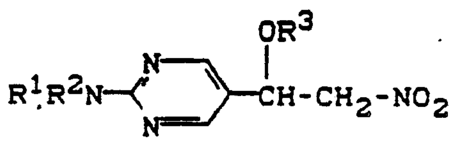

(IX)

in welcher
$R^1$ für Wasserstoff, Alkyl, Acyl steht,
$R^2$ für Wasserstoff, Alkyl steht,
$R^3$ für Wasserstoff, Alkyl, Acyl steht.

14. Verfahren zur Herstellung der Verbindungen der Formel IX gemäß Anspruch 13, dadurch gekennzeichnet, daß man Verbindungen der Formel XIII

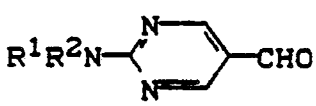

(XIII)

in welcher
$R^1$ und $R^2$ die in Anspruch 13 angegebene Bedeutung haben,
mit Nitromethan umsetzt und gegebenenfalls anschließend alkyliert und acyliert.

15. Neue Verbindungen der Formel X

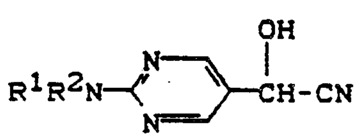

(X)

in welcher

$R^1$ für Wasserstoff, Alkyl, Acyl steht,
$R^2$ für Wasserstoff, Alkyl steht.

16. Verfahren zur Herstellung der Verbindungen der Formel X gemäß (15), dadurch gekennzeichnet, daß man Verbindungen der Formel XIII

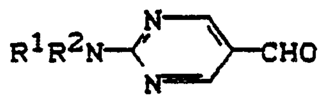

(XIII)

in welcher
$R^1$ und $R^2$ die in Anspruch 15 angegebene Bedeutung haben,
mit Blausäure oder deren Salzen umsetzt.

17. Neue Verbindungen der Formel XIII

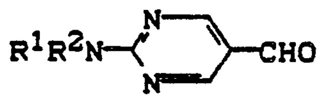

(XIII)

in welcher
$R^1$ für Wasserstoff, Alkyl, Acyl steht,
$R^2$ für Wasserstoff, Alkyl steht.

18. Verfahren zur Herstellung der Verbindungen der Formel XIII gemäß Anspruch 17, dadurch gekennzeichnet, daß man Verbindungen der Formel XIV

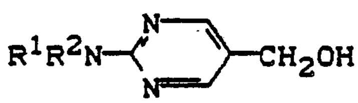

(XIV)

in welcher
$R^1$ und $R^2$ die in Anspruch 17 angegebene Bedeutung haben,
oxidiert.

19. Neue Verbindungen der Formel XIV

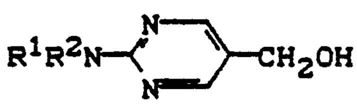

(XIV)

in welcher
$R^1$ für Wasserstoff, Alkyl, Acyl steht,
$R^2$ für Wasserstoff, Alkyl steht.

20. Verfahren zur Herstellung der Verbindungen der Formel XIV gemäß Anspruch 19, dadurch gekennzeichnet, daß man Verbindungen der Formel XV

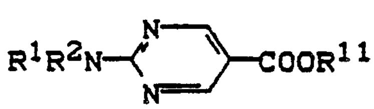

(XV)

in welcher
$R^1$ und $R^2$ die bei (19) angegebene Bedeutung haben, und
$R^{11}$ für H oder Alkyl steht,
reduziert.

21. Mittel zur Leistungsförderung von Tieren, gekennzeichnet durch einen Gehalt an Pyrimidinderivaten der Formel I gemäß Anspruch 1.

22. Tierfutter, Trinkwasser für Tiere, Zusätze für Tierfutter und Trinkwasser für Tiere, gekennzeichnet durch einen Gehalt an Pyrimidinderivaten der Formel I gemäß Anspruch 1.

23. Verwendung von Pyrimidinderivaten der Formel I gemäß Anspruch 1 zur Leistungsförderung von Tieren.

24. Verfahren zur Herstellung von Mitteln zur Leistungsförderung von Tieren, dadurch gekennzeichnet, daß man Pyrimidinderivate der Formel I gemäß Anspruch 1 mit Streck- und/oder Verdünnungsmitteln vermischt.

25. Verfahren zur Herstellung von Tierfutter, Trinkwasser für Tiere oder Zusätze für Tierfutter und Trinkwasser für Tiere, dadurch gekennzeichnet, daß man Pyrimidinderivate der Formel I gemäß Anspruch 1 mit Futtermitteln oder Trinkwasser und gegebenenfalls weiteren Hilfsstoffen vermischt.

26. Verwendung von Pyrimidinderivaten der Formel (I) gemäß Anspruch 1 zur Herstellung von Mitteln zur Leistungsförderung bei Tieren.